# Europäisches Patentamt

## European Patent Office

## Office européen des brevets

(19)

(11) Veröffentlichungsnummer: **0 104 493**
**B1**

(12) **EUROPÄISCHE PATENTSCHRIFT**

(45) Veröffentlichungstag der Patentschrift:
**10.12.86**

(51) Int. Cl.⁴: **A 61 B 5/00**

(21) Anmeldenummer: **83108631.9**

(22) Anmeldetag: **01.09.83**

(54) **Vorrichtung zur Bestimmung von p02-Histogrammen mittels Stichsonde.**

(30) Priorität: **01.09.82 DE 3232455**

(43) Veröffentlichungstag der Anmeldung:
**04.04.84 Patentblatt 84/14**

(45) Bekanntmachung des Hinweises auf die Patenterteilung:
**10.12.86 Patentblatt 86/50**

(84) Benannte Vertragsstaaten:
**AT BE CH DE FR GB LI**

(56) Entgegenhaltungen:
**DE - A - 2 943 958**
**US - A - 4 154 228**

**ANGIOLOGY, Jahrgang 28, Nr. 2, Februar 1977, Seiten 101-108, Baltimore, US, A.M.EHRLY et al.:"Oxygen pressure in ischemic muscle tissue of patients with chronic occlusive arterial diseases"**
**MEDICAL AND BIOLOGICAL ENGINEERING, Band 14, Nr. 2, März 1976, Seiten 159-165, Stevenage, GB, V.A.SPENCE et al.:"Measurement of oxygen tension in human skin"**

(73) Patentinhaber: **GMS, Gesellschaft für medizinische Sondentechnik mbH, Muhliusstrasse 38, D-2300 Kiel 1 (DE)**

(72) Erfinder: **Sorger, Peter, Lohausweg 17, D-4400 Münster (DE)**

(74) Vertreter: **Schaefer, Konrad, Gehölzweg 20, D-2000 Hamburg 70 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Vorrichtung der im Oberbegriff des Anspruches 1 genannten Art.

Zum Stand der Technik wird verwiesen auf den Artikel von D.W. Lübbers «Grundlagen und Bedeutung der lokalen Sauerstoffdruckmessung und des $pO_2$-Histogramms für die Beurteilung der Sauerstoffversorgung der Organe und des Organismus» in A.M. Ehrly «Messung des Gewebesauerstoffdruckes bei Patienten», Verlag Gerhard Witzstrock, 1981, Seiten 11–18. Der Stand der Technik ergibt sich demnach wie folgt:

Verlässliche, für die medizinische Diagnose verwertbare Aussagen über die Sauerstoffversorgung des Gewebes lassen sich nur über die Ermittlung der statistischen Verteilung örtlicher Sauerstoffdruckunterschiede im Gewebe ermitteln. Dazu muss an einer grösseren Zahl von Messorten (üblicherweise wenigstens 100) der lokale Sauerstoffdruck ($pO_2$) bestimmt werden. Nach statistischer Verarbeitung dieser Messwerte ergibt sich das sogenannte $pO_2$-Histogramm, das in einer grafischen Darstellung eine diagnostisch verwertbare Aussage über die vorliegenden Sauerstoffversorgungsverhältnisse liefert. Ein Beispiel ist in der zitierten Arbeit auf Seite 15 angegeben.

Nach der zitierten Arbeit sind Vorrichtungen der eingangs genannten Art bekannt, die gemäss Seite 14, rechte Spalte Mitte, Sondenspitzendurchmesser von unten 1 µm aufweisen. Nach Seite 16, dritter Absatz, ist das Arbeiten mit derartigen Mikrostichsonden jedoch äusserst aufwendig.

Die Nachteile der Mikrostichsonden liegen im wesentlichen in deren Bruchgefahr. Eine Anwendung kommt daher nur in hochspezialisierten wissenschaftlichen Labors, nicht aber in der Klinikroutine in Frage.

Auf Seite 16 im dritten Absatz wird weiterhin darauf verwiesen, dass dickere, bruchsicher mit Metallkanülen ummantelte Sonden bekannt sind, die jedoch in vivo unzuverlässige Messwerte liefern, was vermutlich auf lokale Beeinflussung der Durchblutung zurückzuführen ist. Diese bruchsicheren Sonden sind daher für die vorliegenden Zwecke nicht einsetzbar.

Die Aufgabe der vorliegenden Erfindung besteht darin, eine Vorrichtung der eingangs genannten Art zu schaffen, die verlässliche Ergebnisse bei für den klinischen Betrieb zumutbarer Handhabbarkeit ermöglicht.

Diese Aufgabe wird erfindungsgemäss mit den Merkmalen des Kennzeichnungsteiles des Anspruches 1 gelöst.

Erfindungsgemäss wird eine stabile, bruchsichere und daher einfach handhabbare Sonde verwendet, die in ihrem Durchmesser um wenigstens eine Grössenordnung über dem der Mikrostichsonde liegt. Die Erfindung geht dabei von der Erkenntnis aus, dass nach Einstechen derart dicker Sonden bis zu einem Messort im Gewebe der Sauerstoffdruck am Messort absinkt, was auf Verdrängungskompression des umliegenden Gewebes durch die dicke Sonde zurückzuführen ist.

Dieser Effekt tritt naturgemäss bei den erheblich dünneren Mikrostichsonden (Durchmesser etwa unter 1 µm) nicht auf. Der Einflussbereich um die Sonde, in dem der $pO_2$ absinkt, hängt vom Gewebetyp (in Versuchen ermittelbar) sowie vom Durchmesser der Sonde ab. Es wird daher zwischen zwei Messorten immer so weit verschoben, dass die Sondenmessstelle an einen von der vorherigen Stellung noch unbeeinflussten Ort kommt. Die Erfindung geht weiterhin von der Erkenntnis aus, dass der durch die Sonde hervorgerufene $pO_2$-Abfall mit einer Geschwindigkeit erfolgt, die vom Ort im Gewebe abhängig ist. Die mittlere Abfallzeitkonstante $\tau_g$ ist gewebespezifisch und kann ermittelt werden. Bei ausreichend schnellem Vorschub werden stets unbeeinflusste Messorte erreicht, an denen in kurzem zeitlichem Abstand nach Sondenstillstand der wahre $pO_2$ ermittelt werden kann. Die Zeitkonstante des Ansprechverhaltens der Sonde muss dementsprechend klein sein, um möglichst zu Beginn der durch die Sonde verursachten $pO_2$-Änderung messen zu können, zu einem Zeitpunkt also, an dem noch wenigstens annähernd der ursprüngliche $pO_2$-Wert ermittelt werden kann. Bei entsprechendem Verhältnis der Ansprechzeitkonstante der Vorrichtung im Verhältnis zur Änderungszeitkonstante des Gewebe-$pO_2$ und bei Messung möglichst unmittelbar nach Abwarten der Ansprechzeit der Sonde lässt sich tatsächlich der wahre $pO_2$ des umliegenden Gewebes ermitteln, was nach dem Stand der Technik nicht für möglich gehalten wurde. Die Messstelle der Sonde kann nach einem geeigneten physikalischen Prinzip funktionieren, beispielsweise nach einem optischen Verfahren unter Verwendung von Lichtleitern oder nach einem bekannten polarografischen Verfahren. Bei letzterem können bei der Verschiebungsbewegung der Sonde Störeffekte auftreten, die nach Stillstand etwa innerhalb der abzuwartenden Ansprechzeit der Sonde abklingen und die Messung zum Zeitpunkt der Ermittlung des Messwertes nicht mehr stören. Erfindungsgemäss ergibt sich also eine Vorrichtung, die bei robustem, für Routineanwendung geeignetem Sondenaufbau eine präzise Ermittlung des wahren Gewebe-$pO_2$ und damit die Erstellung eines aussagekräftigen $pO_2$-Histogrammes ermöglicht. Die erfindungsgemässe Vorrichtung ermöglicht aufgrund ihrer einfachen Handhabung sowie der zulässigen raschen Schrittfolge eine sehr schnelle Ermittlung eines kompletten Histogrammes aus den erforderlichen etwa 100 Messstellen, das beispielsweise innerhalb von etwa zwei Minuten ermittelt werden kann. Diese hohe, bisher unerreichte Messgeschwindigkeit ist weitere Voraussetzung für erfolgreiche klinische Anwendung.

Vorteilhaft ist die erfindungsgemässe Vorrichtung durch die Merkmale des Anspruches 2 gekennzeichnet. Bei derartig kleiner Zeitkonstante des Ansprechverhaltens der Vorrichtung ist der Messwert noch eher und damit noch genauer dem ungestörten Gewebe-$pO_2$ entsprechend ermittelbar.

Weiterhin vorteilhaft ist die erfindungsgemässe Vorrichtung durch die Merkmale des Anspruches 3 gekennzeichnet. Ab etwa diesem Durchmesserbereich lässt sich die Sonde der Vorrichtung besonders einfach und messwertstabil herstellen.

Weiterhin vorteilhaft ist die erfindungsgemässe Vorrichtung durch die Merkmale des Anspruches 4 gekennzeichnet. Die Sonde lässt sich auf diese Weise einfach fertigen bei sehr hoher mechanischer Stabilität, insbesondere Bruchfestigkeit.

Weiterhin vorteilhaft ist die erfindungsgemässe Vorrichtung durch die Merkmale des Anspruches 5 gekennzeichnet. Dieser Sondentyp mit polarografischer Messstelle hat sich bei der Anmelderin als hervorragend geeignet herausgestellt und zeichnet sich insbesondere durch geringe Herstellungskosten aus, was die klinisch vorteilhafte Einmalverwendung ermöglicht. Aufgrund der geringen aktiven Metalloberfläche ergeben sich günstige polarografische Daten.

Eine polarografische Makrostichsonde, die vorteilhaft auch für die vorstehend genannten Zwecke verwendbar ist, ist durch die Merkmale des Anspruches 6 gekennzeichnet. Die Verwendung von Vergussmasse erlaubt günstige Herstellung, insbesondere bei Ausbildung gemäss Anspruch 4.

Dabei ist vorteilhaft gemäss Anspruch 7 die Metallfilmoberfläche gegenüber der Schlifffläche in einem Rezess zurückgezogen. Dadurch wird die reproduzierbare Herstellung von driftarmen Sonden mit gleichmässigen polarografischen Daten ermöglicht, die sich insbesondere durch äusserst kurze, für die erfindungsgemässe Verwendung notwendige Ansprechzeiten mit einer Zeitkonstante von weit unterhalb einer Sekunde auszeichnen.

Schliesslich ist die erfindungsgemässe Vorrichtung durch die Merkmale des Anspruches 8 gekennzeichnet. Eine derartige Sonde ist für die Routineunteruschung in der Skelettmuskulatur des Menschen besonders geeignet, z.B. zur Untersuchung des sogenannten Raucherbeines.

In den Zeichnungen ist die Erfindung beispielsweise und schematisch dargestellt. Es zeigen:

Fig. 1 die schematische Gesamtdarstellung einer erfindungsgemässen Vorrichtung,

Fig. 2a einen in Sondenachse gelegten Schnitt durch einen Messort im Gewebe,

Fig. 2b einen Schnitt nach Linie 200 in Fig. 2a,

Fig. 3a bis 3e übereinander ausgerichtete zeitliche Verläufe von Parametern zur Erläuterung der Entstehung des in Fig. 3e dargestellten Messignales der Sondenmessstelle,

Fig. 4 in zeitlich übereinander ausgerichteter Darstellung zum Messignal die Darstellung unterschiedlicher Steuerungsabläufe in der Vorrichtung gemäss Fig. 1 und

Fig. 5 eine perspektivische Ansicht der Sondenmessstelle und

Fig. 6 einen vergrösserten Achsschnitt durch die Messstelle gemäss Fläche 57 in Fig. 5.

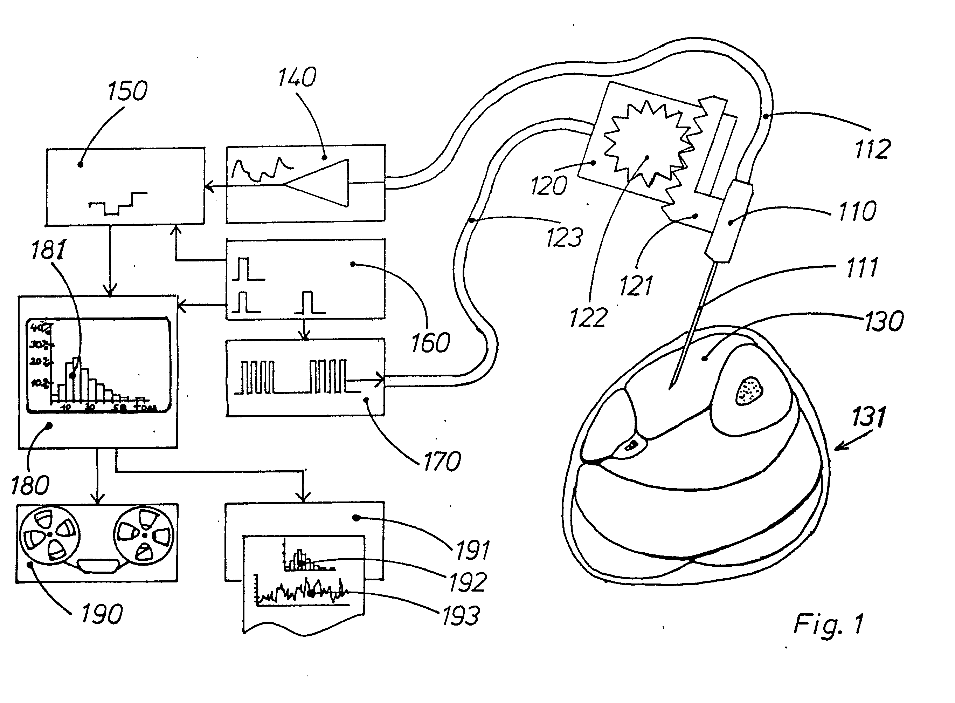

Fig. 1 zeigt in schematischer Gesamtdarstellung eine erfindungsgemässe Vorrichtung im klinischen Einsatz.

An einem Sondenträger 110 ist eine Stichsonde 111 befestigt. Die in der Sondenspitze angeordnete polarografische Messstelle ist über ein Sondenanschlusskabel 112 mit einer im Blockschaltbild dargestellten Messignalauswerteinrichtung verbunden.

Ein stationär z.B. an einem nicht dargestellten Stativ angeordneter Sondenantrieb 120 treibt mittels eines elektronisch ansteuerbaren Motors den Sondenträger 110 in Achsrichtung der Sonde 111 an. Im dargestellten Ausführungsbeispiel ist dies schematisch mit Zahnstange 121 und Zahnrad 122 dargestellt. Energieversorgung und Steuerung des Antriebes 120 erfolgt über ein Anschlusskabel 123.

In Fig. 1 ist eine typische Anwendung für klinische Diagnosezwecke dargestellt. Die Sonde 111 ist in den musculus tibialis anterior 130 eines menschlichen Unterschenkels 131 eingestochen, welcher in der Figur im Schnitt dargestellt ist.

Die im Blockschaltbild in Fig. 1 dargestellte Messignalauswerteinrichtung weist einen an die Messstelle der Sonde über das Kabel 112 angeschlossenen Messignalverstärker 140 auf, von dem das verstärkte Messignal einer Einrichtung 150 zur Bestimmung des wahren Gewebe-$pO_2$ zugeführt wird und die von einem Zeitsteuergerät 160 gesteuert wird. Dieses gibt ausserdem Steuerimpulse an eine Motorantriebssteuerung 170, die über das Kabel 123 an den Antrieb 120 angeschlossen ist, und an einen Histogrammrechner 180 mit Sichtschirm 181 zur Darstellung des errechneten Histogrammes. Von dem Rechner 180 werden ferner ein Datenspeichergerät 190 sowie ein Datendrucker 191 angesteuert, auf dem je nach Ausbildung des Rechners 180 das Histogramm 192 sowie gegebenenfalls zusätzlich eine Darstellung 193 des $pO_2$-Profils im durchfahrenen Gewebe auf dem dargestellten Papierstreifen ausgedruckt werden können.

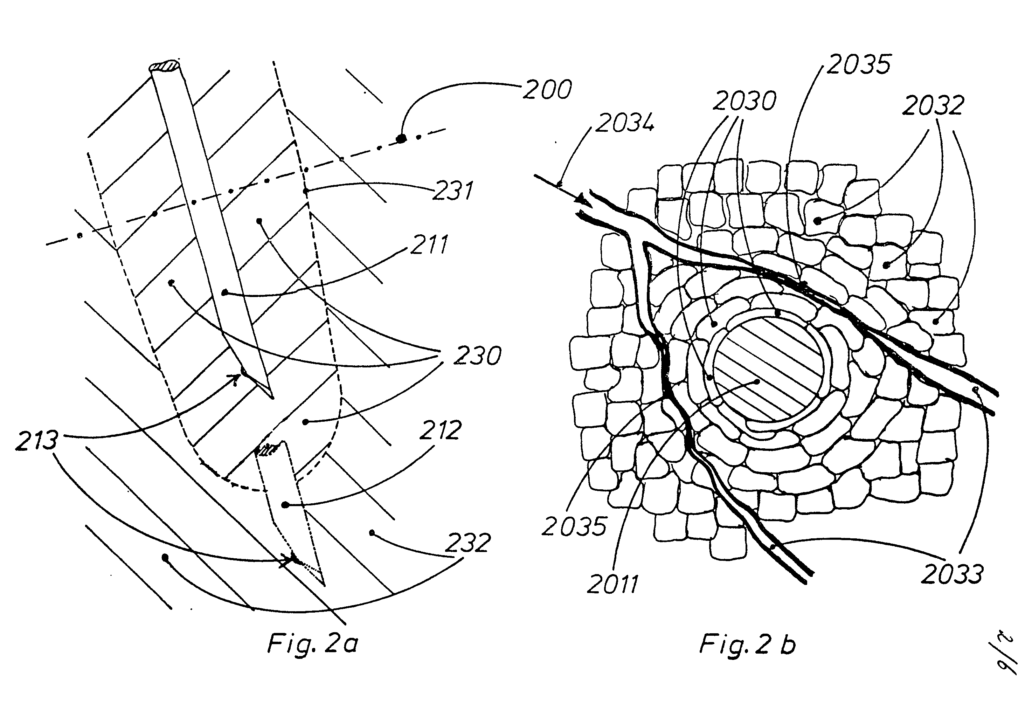

Fig. 2a zeigt in starker Vergrösserung die Spitze der Sonde 111 in einer Stellung 211 sowie in einer um einen Schritt des Antriebes 120 vorgeschobenen Stellung 212. Die Darstellung der Fig. 2a zeigt eine im Inneren einer Metallkanüle angeordnete Sonde mit dem typischen Spitzenschliff einer dünnen Metallkanüle, die einen Aussendurchmesser im Bereich weniger 100 µm aufweist. Bei 213 ist in der Schlifffläche der Kanüle die Messstelle angeordnet.

Die Darstellung der Fig. 2a zeigt die Sondenspitze eingestochen in ein schraffiert dargestelltes Gewebe. Bei den erfindungsgemäss verwendeten Sonden mit Durchmessern über 10 µm, vorzugsweise über 100 µm, wobei der Durchmesser bis über 500 µm betragen kann, beeinflusst die Sonde durch Verdrängung und dadurch hervorgerufene Kompression in einer Kompressionszone 230, die etwa durch die Grenzlinie 231 begrenzt ist, das umliegende Gewebe, wobei ausserhalb der Grenzlinie 231 liegendes Gewebe 232 von der Kompression unbeeinflusst bleibt. Die Kompressionszone 230 hat einen Radius, der in der Grössenordnung des Sondendurchmessers liegt, in der Darstellung der Fig. 2 beträgt der Kompressionszonenradius etwa das Dreifache des Durchmessers. Der Kompressionszonenradius hängt von der Kompressionsempfindlichkeit des untersuchten Gewebetyps ab und kann für jeden Gewebetyp ermittelt und in einer Tabelle dem Arzt zur Verfügung gestellt werden.

Fig. 2b zeigt einen Schnitt senkrecht zur Darstellung der Fig. 2a. 2011 zeigt die Sonde im Querschnitt bei der Schnittlinie 200 in Fig. 2a. Mit 2030 sind in der Kompressionszone 230 liegende, durch Kompression stark deformierte und gequetschte Gewebezellen dargestellt. Ausserhalb des Kompressionsbereiches, also ausserhalb der Grenzlinie 231 liegende Zellen 2032 sind nicht komprimiert. Ein das Gewebe versorgender Blutstrom 2034 durchläuft Blutgefässe 2033, die im Bereich der Kompressionszone 230, wie die Fig. 2b zeigt, im Querschnitt verringert sind. Die Blutversorgung und damit die Sauerstoffversorgung ist also durch die Verengung der Blutgefässe bei 2035 im Bereich der Kompressionszone herabgesetzt.

Nach Einstechen der Sondenspitze in die Lage 211 (Fig. 2a) sinkt somit in der Kompressionszone 230 der Sauerstoffdruck ab. Die Schrittweite zwischen zwei Messorten muss also so weit sein, dass zwischen der Sondenspitzenstellung 211 an einem Messort und der Stellung 212 an dem nächsten Messort ein Schritt liegt, der die Messstelle 213 aus der durch die vorhergehende Stellung 211 erzeugten Kompressionszone 230 herausbringt. Die Schrittweite muss mindestens den Radius der Kompressionszone betragen. Liegt die Messstelle 213 wesentlich hinter der Sondenspitze, so ist dieser Abstand bei der Schrittweite noch hinzuzählen. Über den vom Durchmesser der Sonde abhängigen Kompressionszonenradius hängt die Schrittweite von der jeweiligen Struktur des vorliegenden Gewebetypes sowie ausserdem von der Sondendicke ab. In der Skelettmuskulatur des Menschen, also beispielsweise in dem in Fig. 1 erläuterten Anwendungsfall und bei einem Sondendurchmesser von ca. 200 bis 500 µm, bei einem Einstichwinkel von weniger als 45° zur Muskelfaserlängsachse und bei einer Sondenspitzengeometrie gemäss Fig. 2a ist eine schrittweite von über ca. 800 µm erforderlich.

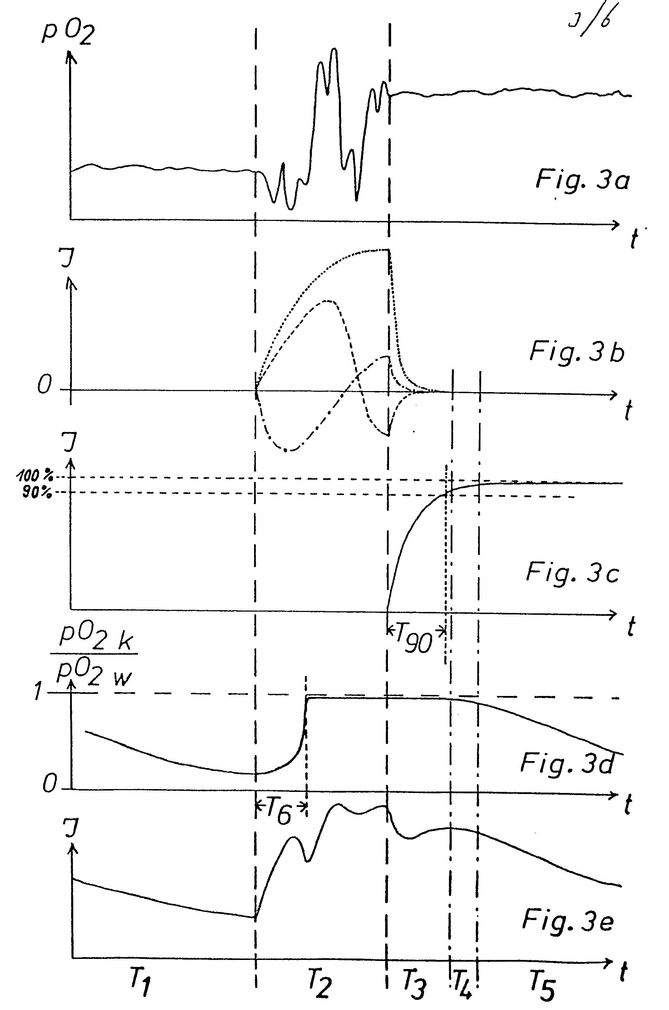

Die Fig. 3a bis 3e zeigen zeitliche Verläufe von Parametern über der Zeitachse t als Abzisse. Innerhalb des Zeitabschnittes $T_1$ steht die Sondenspitze an einem Messort, beispielsweise gemäss Stellung 211 (Fig. 2a). Innerhalb des Zeitabschnittes $T_2$ findet eine Schrittbewegung der Sonde von einem Messort zum nächsten statt. Bei $T_3$, $T_4$, $T_5$ steht die Sonde am nächsten Messort (212 gemäss Fig. 2a).

In Fig. 3a sind die Sauerstoffdruckwerte ($pO_2$) dargestellt, die idealerweise entsprechend den wahren $pO_2$-Werten mit einer idealen Sonde, z.B. einer kompressionseffektfreien Mikrostichsonde, ermittelbar wären. Am ersten Messort bei $T_1$ ergibt sich ein bestimmter $pO_2$. Während der Schrittzeit $T_2$ werden Gebiete unterschiedlichen Sauerstoffdruckes durchfahren, und bei $T_3$, $T_4$, $T_5$ ergibt sich ein neuer $pO_2$-Wert. Vor und nach der Schrittbewegung bleiben die $pO_2$-Werte konstant.

Fig. 3b zeigt in drei unterschiedlichen typischen Beispielen Störeffekte, die bei polarografischen Sonden während der Sondenbewegung, also innerhalb $T_2$, auftreten und die nach Sondenstillstand schnell abklingen. Es ist hierbei angenommen, dass die Sonde in einem Gebiet mit ortsunabhängig konstantem $pO_2$ verschoben wird. Das Störsignal ist als Messwertanteil I des Polarografierstromes aufgetragen.

Fig. 3c zeigt das Ansprechverhalten der Vorrichtung, das im wesentlichen durch deren langsamste Komponente, nämlich die Messstelle 213, bestimmt ist. Das Stromsignal I der Messstelle ist nach einem $pO_2$-Sprung von 0 auf 100% am Beginn von $T_3$ dargestellt. Die Vorrichtung zeigt 90% des wahren Wertes nach der Zeit $T_{90}$ an. Dies entspricht etwa $3\tau_V$, wobei $\tau_V$ die Zeitkonstante des Ansprechverhaltens der Vorrichtung ist.

Fig. 3d zeigt die durch den Kompressionseffekt bedingte Sauerstoffverringerung im die Sondenspitze umgebenden Kompressionsbereich 230 (Fig. 2a). Es ist dargestellt das Verhältnis von durch Kompression gestörtem Sauerstoffdruck $pO_{2k}$ zu ungestörtem wahren $pO_{2w}$. Nach Beginn der Schrittzeit $T_2$ verlässt die Messstelle etwa nach Ablauf der Zeit $T_6$ den Kompressionsbereich 230 und durchläuft bis zum Ende der Schrittzeit $T_2$ ungestörte Gewebebereiche. Nach Stillstand der Sonde am nächsten Messort, also bei Beginn von $T_3$ beginnt durch Kompressionseinwirkung der Sauerstoffdruck im Bereich der Sondenspitze, also im Kompressionsbereich 230, wieder abzusinken mit einer Zeitkonstante $\tau_g$, was der abfallenden Kurve im Bereich $T_3$, $T_4$, $T_5$ entspricht. Der Abfall erfolgt zunächst schwach und sodann stärker, so dass zu Anfang des Abfalles noch der wahre $pO_2$-Wert vorliegt ($pO_{2k}/pO_{2w}$ etwa = 1). In Fig. 3d ist wiederum vorausgesetzt, dass die Sonde Gebiete gleichen Sauerstoffdruckes durchläuft.

In Fig. 3e ist ein Beispiel eines tatsächlich sich ergebenden Messignales (Polarografierstrom I) dargestellt. Dieser Verlauf ergibt sich im wesentlichen durch Überlagerung der Effekte gemäss den Fig. 3b, 3c und 3d. Im Bereich $T_1$ ist der abfallende Teil der Kompressionskurve gemäss Fig. 3d ersichtlich. Der Schrittbereich $T_2$ ist im wesentlichen durch die geschwindigkeitsabhängigen Störeffekte gemäss Fig. 3b und die lokalen Sauerstoffunterschiede gemäss Fig. 3a bestimmt. Nach Sondenstillstand am Beginn von $T_3$ ist zunächst das Abklingen der Störeffekte gemäss Fig. 3b sowie das Sondenansprechverhalten gemäss Fig. 3c erkennbar. Im Bereich $T_4$ entspricht das Messsignal annähernd dem wahren ungestörten Gewebe-$pO_2$, während es im Bereich $T_5$ abfällt, (wie bei $T_1$).

Im Bereich $T_4$ sind die Störeffekte gemäss Fig. 3b abgeklungen. Die Sonde hat gemäss Fig. 3c ihre Ansprechzeit $T_{90}$ hinter sich und zeigt annähernd 100% an. Gemäss Fig. 3d ist der kompressionsabhängige Sauerstoffdruck noch nicht wesentlich abgesunken. Der Bereich $T_4$ eignet sich

also als Messfenster zur Ermittlung eines Messwertes, der mit grosser Annäherung dem wahren Sauerstoffdruck am Messort entspricht und zur Erstellung des Histogrammes verwertet werden kann.

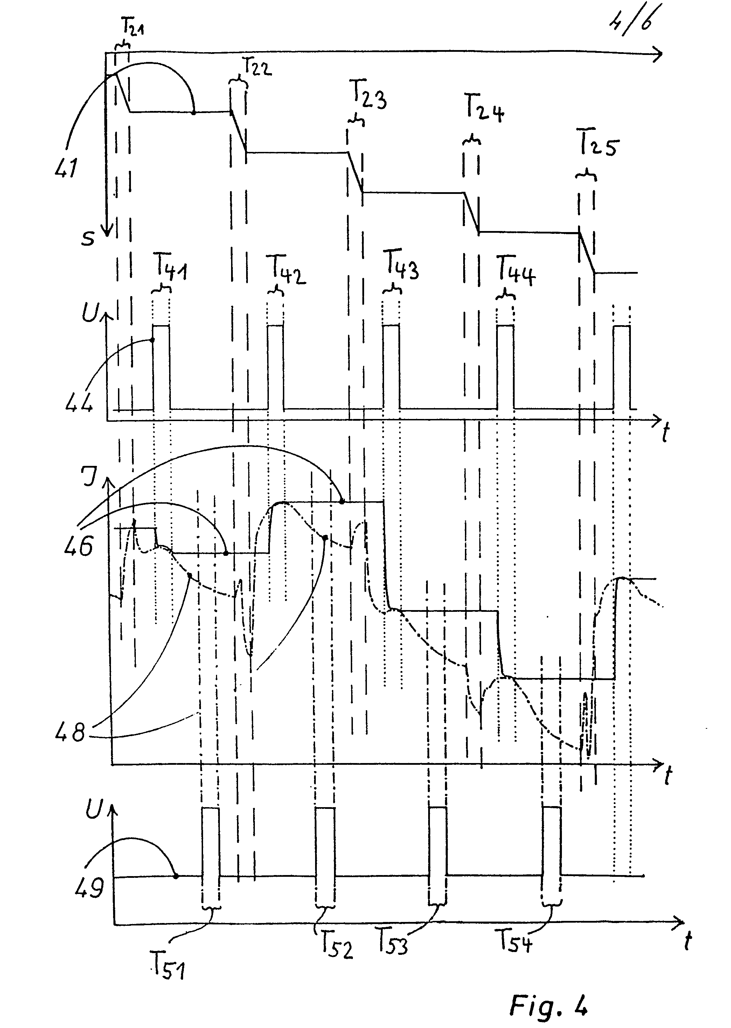

Fig. 4 zeigt den Zeitablauf in der aus Fig. 1 ersichtlichen Messignalauswerteinrichtung, der durch das Zeitsteuergerät 160 mit entsprechenden Impulsen gesteuert wird. In synchroner Übereinanderstellung auf der Zeitachse t sind mehrere Parameter angegeben.

In der obersten Darstellung ist der Weg S des Sondenvorschubes angegeben. Die Ortskurve 41 der Sondenspitze zeigt die Verweilzeiten und die Bewegungszeiten mit linearer Vorschubbewegung. Die Darstellung I (Polarografierstrom) zeigt das sich tatsächlich ergebende Messignal 48 gemäss Fig. 3e. In Fig. 4 sind fünf aufeinanderfolgende Schritte dargestellt, wobei an den Messorten jeweils unterschiedliche pO$_2$-Werte ermittelt werden. Die Kurve 44 zeigt diejenige Ausgangsspannung U des Zeitsteuergerätes 160, die der Einrichtung 150 zur Auswertung des Messignales zugeführt wird. Mit den Torimpulsen 44 wird während der Zeit T$_4$ (Fig. 3e) ein Messfenster geöffnet.

Die Messignalkurve 48 zeigt, jeweils den typischen Verlauf gemäss Fig. 3e im Bereich der Schrittzeiten T$_{21}$ bis T$_{25}$. Dazwischenliegend ergibt sich der Kompressionsabfall, der bei Fig. 3e im Bereich T$_1$ und T$_5$ zu beobachten ist. Die Messfenstersteuerimpulse des Steuersignales 44 liegen bei T$_{41}$ bis T$_{45}$. Am Ausgang der Einrichtung 150 zur Bestimmung des wahren Gewebe-pO$_2$ liegt ein etwa treppenförmig verlaufendes Signal 46, das von einem Messfenster bis zum anderen konstant bleibt und dem jeweils vorher ermittelten Messwert an einem Messort entspricht. Bei den Zeiten T$_{51}$, T$_{52}$, T$_{53}$, T$_{54}$ erfolgt mit Steuerimpulsen 49 vom Zeitsteuergerät 160 auf die Einrichtung 150 die Übertragung dieser Werte zum Rechner 180, der diese zur Ermittlung des Histogrammes benötigt.

Es sei nun noch einmal auf einige verwendete Begriffe etwas genauer wie folgt eingegangen:

Die Literatur gibt bei für die vorliegenden Zwecke bisher allein verwendeten Mikrosonden Vorschubgeschwindigkeiten von wenigen 100 µm pro Minute an. Bei dünnen kompressionseffektfreien Mikrosonden sind derart langsame Vorschubgeschwindigkeiten möglich. Zur wesentlichen erfindungsgemässen Erkenntnis gehört es, dass erfindungsgemäss verwendete dicke Sonden Kompressionseffekte ergeben, die zu einem pO$_2$-Abfall im umliegenden Gewebe führen. Dieser erfolgt mit einer gewissen mittleren Zeikonstante τ$_g$. Bewegt man die Sonde zu langsam, so verlässt die Messstelle 213 nie die sich um die Sondenspitze ausbreitende Kompressionszone 230. Die Vorschubgeschwindigkeit muss also um wenigstens ein bis zwei Grössenordnungen höher sein als die Abfallgeschwindigkeit des Gewebe-pO$_2$. Die Schrittzeit T$_2$ ist daher kleiner als ein Zehntel τ$_g$ oder vorzugsweise kleiner als ein Hundertstel τ$_g$ zu wählen.

Es ist hier noch zu Fig. 3d anzumerken, dass dort T$_6$ zur besseren zeichnerischen Darstellung erheblich vergrössert dargestellt ist. Weiterhin ist zu dieser Darstellung noch zu bemerken, dass nach Sondenstillstand der Gewebe-pO$_2$ nicht sofort abfällt, sondern noch über eine gewisse Zeit konstant bleibt (etwa T$_3$), bevor der Abfall einsetzt. Dies kann beispielsweise auf Sauerstoffspeichereffekte im Gewebe zurückgeführt werden.

Erfindungsgemäss verwendete Bewegungsgeschwindigkeiten für die Sonde liegen beispielsweise bei 600 000 µm pro Minute, also über drei Grössenordnungen höher als aus dem Stand der Technik für Mikrosonden bekannt.

Zur Gewebe-pO$_2$-Abfallgeschwindigkeit, die in Vorstehendem durch die Zeitkonstante τ$_g$ gekennzeichnet wurde, ist noch zu bemerken, dass in einem vorgegebenen Gewebe je nach vorliegender Mikrostruktur τ$_g$ stark variieren kann. In den vorstehenden Erläuterungen ist mit τ$_a$ stets der Mittelwert angegeben, der gewebespezifisch ermittelbar ist. Wenn erfindungsgemäss der Messwert spätestens nach ein Zehntel τ$_g$ nach Sondenstillstand ermittelt wird, so liegen nur an wenigen Messorten erheblich schnellere pO$_2$-Abfallzeiten vor, was an diesen Messorten zu Messwertverfälschungen führt. Die Anzahl der falschen Messwerte liegt dann etwa unter 1%, was bei der statistischen Auswertung zum pO$_2$-Histogramm nicht mehr verfälschend wirkt.

Im folgenden wird eine Ausführungsform der Sonde 111 beschrieben, die für die erfindungsgemässe Vorrichtung besonders gut geeignet ist. Diese Ausführungsform der Sonde ist jedoch nicht auf den vorliegenden Anwendungsfall der Makrosonde beschränkt, sondern eignet sich im beschriebenen Aufbau der polarografisch aktiven Messfläche insbesondere auch für Mikrosonden erheblich geringeren Durchmessers sowie für andere Sondentypen, beispielsweise für Aufsetzsonden.

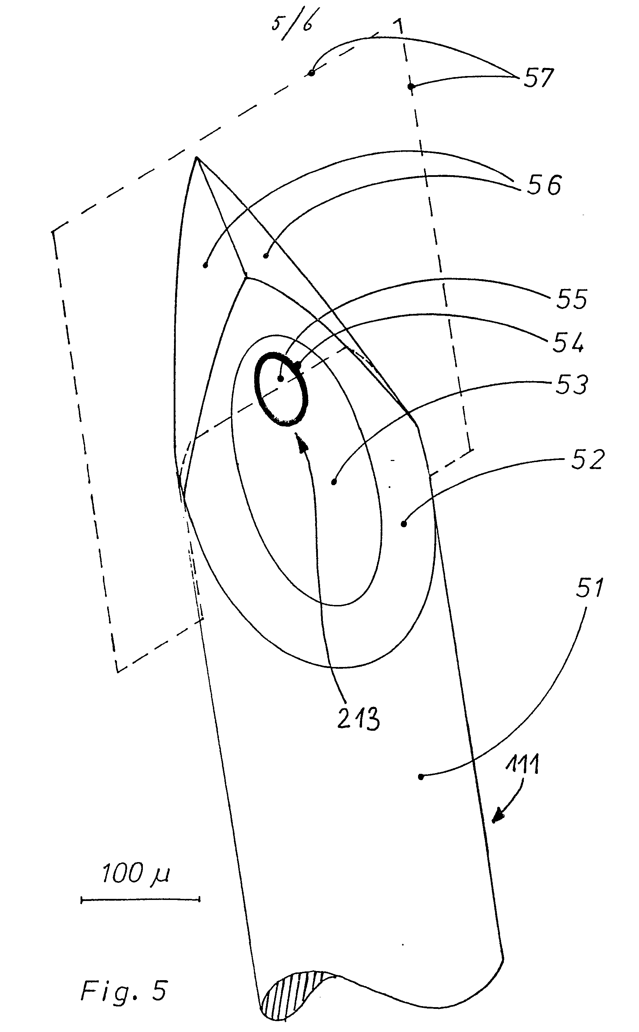

Fig. 5 zeigt perspektivisch die Spitze der in den Fig. 1 und 2a dargestellten Sonde 111.

Ein Stahlmantel 51 mit geschliffener Spitze wird beispielsweise in Form einer handelsüblichen Spritzenkanüle verwendet, die einen Aussendurchmesser im Bereich einiger 100 µm aufweist. Die Schlifffläche weist einen Hauptschliff 52 sowie Lanzettschliffflächen 56 auf und eignet sich daher vorzüglich zum zerstörungsfreien Einstechen in das zu untersuchende Gewebe.

Die Messstelle 213, die im beschriebenen Ausführungsbeispiel als polarografische Messstelle ausgebildet ist, weist einen in der Ebene des Schliffes 52 liegenden Elektrodenring 54 auf, der im Ringinneren einen Isolierkörper 55 aufweist. Ausserhalb des Elektrodenringes 54 ist der Raum innerhalb des Stahlmantels 51 mit Vergussmasse 53 ausgefüllt, die ebenfalls elektrisch hochisolierend ausgebildet ist.

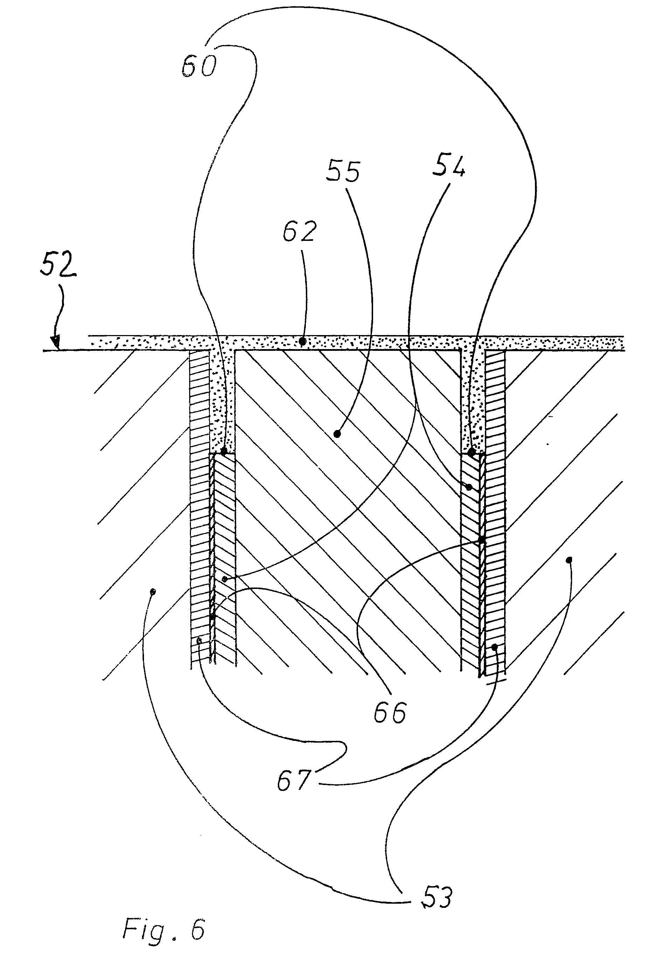

Einzelheiten der Messstelle 213 ergeben sich aus der vergrösserten Schnittdarstellung der Fig. 6.

Der Isolierkörper 55 ist als langgestreckte Faser, beispielsweise Quarzfaser, ausgebildet. Auf deren im wesentlichen zylindrischer Oberfläche ist der Elektrodenring 54 in Form eines Metallfilmes aufgebracht, der sich im wesentlichen über die Länge der Faser bis zu deren rückwärtigem Ende erstreckt, wo die Elektrode direkt am Film kontaktiert und an das Kabel 112 angeschlossen werden kann.

Auf dem Film 54, der aus für die vorliegenden Zwecke, also die $pO_2$-Polarografie geeignetem Metall besteht, ist eine Chromschicht 66 aufgebracht. Auf diese folgt eine Isolatorschicht 67. Bei diesem Schichtaufbau sorgt die Chromschicht 66 für spaltfreie Abdichtung zur Isolatorschicht 67. Erfolgt bei Wasserangriff und angelegter Polarografierspannung ein chemischer Angriff, so verhindert eine Passivierung der Chromschicht sehr schnell weiteren Angriff. Die polarografischen Daten der Messstelle bleiben daher auch bei längerem Einsatz konstant.

Die beschriebene Anordnung einschliesslich der Isolierschicht 67 wird bei der Herstellung im Stahlmantel 51 angeordnet und mit der hochisolierenden Vergussmasse 53 befestigt. Anschliessend wird in der Schlifffläche 52 (Fig. 5) geschliffen. Die vordere Schnittfläche des Elektrodenfilmes 54 mit der Schlifffläche 52 bildet dann den in Fig. 5 dargestellten Elektrodenring. Es muss eine Diffusionsmembran 62, die aus Fig. 6 ersichtlich ist, über der Anordnung aufgebracht werden, um eine funktionierende polarografische Messstelle zu erhalten.

Der Durchmesser des Elektrodenringes bzw. -filmes 54, der die eigentliche Messstelle ausbildet, ist vorzugsweise sehr gering und liegt etwa im Abmessungsbereich von Mikrostichsonden, also bei oder unter etwa 1 μm. Der restliche Sondendurchmesser ist im wesentlichen zur Erhöhung der mechanischen Stabilität erforderlich. Der Ringdurchmesser kann jedoch auch, wie aus Fig. 5 ersichtlich, um Grössenordnungen höher liegen. Über entsprechende Ausbildung der Dicke des Elektrodenfilmes 54 kann dessen vordere polarografierend wirkende freie Oberfläche dennoch so klein gewählt werden, dass der Polarografierstrom kleingehalten wird. Dadurch ergibt sich ein niedriger Sauerstoffverbrauch beim Polarografierprozess, also eine geringe Rühreffektabhängigkeit. Die Diffusionsmembran 62 kann dünn gehalten werden. Dadurch ergeben sich kurze Diffusionswege und demzufolge eine hohe Ansprechgeschwindigkeit, also ein kleines $\tau_v$.

Aus Fig. 6 ist zu ersehen, dass im bevorzugten Ausführungsbeispiel die polarografierende Frontfläche 60 des Elektrodenfilmes 54 gegenüber der Schlifffläche 52 zurückgezogen ist. Es bildet sich zwischen der Ringfläche 60 und der Schlifffläche 52 ein Rezessspalt aus, der beim Aufbringen der Membran 62 mit Membranmaterial gefüllt wird.

Die Herstellung des Rezesskanales kann durch Ätzen des Metallfilmes 54 unter die Oberfläche hergestellt werden. Dabei ergibt sich ein erster Vorteil bereits daraus, dass durch den Ätzvorgang die Oberfläche 60 vergleichmässigt und insbesondere verkleinert wird. Etwa über die Schlifffläche 52 vorstehende Filmstücke, die die polarografierende Elektrodenoberfläche vergrössern, werden beseitigt. Exemplarstreuungen in der Serienfertigung werden dadurch verringert, und es wird die polarografisch aktive Elektrodenoberfläche verkleinert.

Ein weiterer Vorteil des Rezessspaltes über der polarografisch aktiven Frontfläche 60 des Filmes 54 liegt darin, dass er auf geometrische Weise die Sauerstoffdiffusion zur polarografischen Ringfläche beeinflusst. Es ergibt sich ein zusätzlicher geometrisch bedingter Gasdiffusionswiderstand. Die Membran 62 kann weiter bis annähernd auf null verringert werden, so dass Membranmaterial nur im Rezessspalt sitzt.

Es hat sich herausgestellt, dass durch Variieren der Rezesstiefe, also des Abstandes zwischen der Fläche 60 und der Schlifffläche 52, ein Optimum der Ansprechgeschwindigkeit bei gleichbleibendem Rühreffekt erhalten werden kann. Auf diese Weise lassen sich also ausserordentlich schnelle, von Exemplarstreuungen fast freie Sonden mit niedrigem Rühreffekt bauen.

Es hat sich herausgestellt, dass die optimale Rezesstiefe etwa in der Grössenordnung der Dicke des Elektrodenfilmes 54 liegt. Das erwähnte Optimum der Ansprechgeschwindigkeit bei genügender Rühreffektfreiheit hat sich bei einer Rezesstiefe von etwa dem 2,5fachen der Filmdicke herausgestellt.

**Patentansprüche**

1. Vorrichtung zur Bestimmung von $pO_2$-Histogrammen im lebenden Gewebe mit einer Stichsonde, die im distalen Bereich eine $pO_2$-Messstelle trägt und von einem Antrieb in Achsrichtung zu einzelnen Messorten bewegbar ist, wobei die Messstelle an eine Messignalausworteinrichtung angeschlossen ist, gekennzeichnet durch die folgenden Merkmale:

a) der Aussendurchmesser der Sonde (111, 211) liegt im Bereich der Messstelle (213) über ca. 10 μm,

b) der Antrieb (120) führt zwischen den Messorten (211, 212) Schritte aus, die grösser sind als der Abstand Messstelle-Sondenspitze plus einer gewebetypabhängigen Strecke (231), die in der Grössenordnung des Sondendurchmessers im distalen Bereich liegt,

c) die Zeitkonstante ($\tau_v$) des Ansprechverhaltens der Vorrichtung einschliesslich der Messstelle auf $pO_2$-Änderungen ist wenigstens etwa 30 mal kleiner als die Mittlere Zeitkonstante ($\tau_g$) des Abfalles des interstitiellen Gewebe-$pO_2$ nach Unterbrechung der Sauerstoffversorgung,

d) die Auswerteinrichtung (140, 150) bestimmt am Messort einen Messwert zu einem Zeitpunkt ($T_4$), der zwischen etwa $3\tau_v$ und etwa $^1/_{10}\tau_g$ nach dem Zeitpunkt der Beendigung der Vorschubbewegung ($T_2$) liegt und

e) die Schrittzeit ($T_2$) der Sondenspitze ist um wenigstens ein bis zwei Grössenordnungen kleiner als die Zeitkonstante ($\tau_g$) des Gewebe-$pO_2$-Abfalles.

2. Vorrichtung nach Anspruch 1, dadurch ge-

kennzeichnet, dass das Verhältnis $\tau_g/\tau_v$ über ca. 100 liegt.

3. Vorrichtung nach einem der vorhergehenden Ansprüche, dadurch gekennzeichnet, dass der Sondendurchmesser im distalen Bereich (211) über ca. 100 μm liegt.

4. Vorrichtung nach einem der vorhergehenden Ansprüche, dadurch gekennzeichnet, dass die Messstelle (213) in der Schlifföffnung einer Metallkanüle befestigt ist.

5. Vorrichtung nach einem der vorhergehenden Ansprüche mit polarografischer Messstelle, dadurch gekennzeichnet, dass die Messstelle als Schlifffläche (52) eines zwischen isolierenden Medien (55, 53) eingebetteten Metallfilmes (54), der von einer diffusionsdurchlässigen Membran (62) überzogen ist, ausgebildet ist.

6. Vorrichtung nach Anspruch 5, dadurch gekennzeichnet, dass der Film (54) auf einem Faserkern (55) ausgebildet und von Vergussmasse (53) umgeben ist.

7. Vorrichtung nach einem der Ansprüche 5 oder 6, dadurch gekennzeichnet, dass die Oberfläche (60) des Metallfilmes (54) um einen Betrag in der Grössenordnung der Filmdicke unterhalb der Schlifffläche (52) liegt.

8. Vorrichtung nach einem der vorhergehenden Ansprüche zur Bestimmung in der Skelettmuskulatur des Menschen, dadurch gekennzeichnet, dass der Sondendurchmesser ca. 200 bis 500 μm und die Schrittweite über ca. 400 μm betragen, dass $\tau_v$ kleiner als etwa 150 ms ist und der Messwert nicht später als ca. 1,5 s nach Bewegungsende ermittelt wird.

**Revendications**

1. Appareil pour la formation d'histogrammes de $pO_2$ dans du tissu vivant à l'aide d'une sonde à aiguille pénétrante, qui porte dans la zone distale un poste de mesure de $pO_2$ et qu'un entraînement peut déplacer en direction axiale vers divers endroits de mesure, le poste de mesure étant relié à une unité d'exploitation des signaux de mesure, appareil caractérisé par les particularités suivantes:

a) le diamètre externe de la sonde (111, 211) se situe dans la région du poste de mesure (213) au-delà d'environ 10 μm,

b) l'entraînement (120) exécute entre les endroits (211, 212) de mesure des pas qui sont supérieurs à la distance poste de mesure – pointe de sonde plus un trajet (213) dépendant du type de tissu, distance qui correspond à l'ordre de grandeur du diamètre de la sonde dans la région distale,

c) la constante de temps ($\tau_v$) du profil de réponse de l'appareil, y compris le poste de mesure, pour des variations de $pO_2$ est inférieure d'environ 30 fois à la constante moyenne de temps ($\tau_g$) de la diminution de la pression $pO_2$ dans les interstices du tissu, après interruption de l'alimentation en oxygène,

d) l'unité d'exploitation (140, 150) détermine à l'endroit de la mesure une valeur de mesure à un instant ($T_4$) qui se situe entre environ $3\tau_v$ et environ $^1/_{10}\tau_g$ après l'instant d'achèvement du mouvement d'avance ($T_2$), et

c) le temps ($T_2$) d'un pas de la pointe de sonde est inférieur d'au moins un à deux ordres de grandeurs à la constante de temps ($\tau_g$) de diminution de la pression $pO_2$ du tissu.

2. Appareil selon la revendication 1, caractérisé en ce que le rapport $\tau_g/\tau_v$ est supérieur à environ 100.

3. Appareil selon l'une des revendications précédentes, caractérisé en ce que le diamètre de sonde est, dans la région distale (211), supérieur à environ 100 μm.

4. Appareil selon l'une des revendications précédentes, caractérisé en ce que le poste de mesure (213) est fixé dans l'orifice de polissage d'une canule métallique.

5. Appareil selon l'une des revendications précédentes, comportant un poste de mesure polarographique, appareil caractérisé en ce que le poste de mesure est réalisé sous forme d'une surface polie (52) d'une pellicule métallique (54) noyée entre des milieux isolants (55, 53), et qui est revêtue d'une membrane (62) perméable à une diffusion.

6. Appareil selon la revendication 5, caractérisé en ce que la pellicule (54) est formée sur un noyau de fibres (55) et est entourée d'une composition (53) de remplissage.

7. Appareil selon l'une des revendications 5 ou 6, caractérisé en ce que la surface (60) de la pellicule de métal (54) se trouve au-dessous de la surface polie (52) à une distance correspondant à l'ordre de grandeur de l'épaisseur de pellicule.

8. Appareil selon l'une des revendications précédentes pour une mesure de détermination dans les muscles moteurs (muscles attachés au squelette) des êtres humains, appareil caractérisé en ce que le diamètre de sonde est d'environ 200 à 500 μm et la largeur de pas est supérieure à environ 400 μm, en ce que $\tau_v$ est inférieur à environ 150 ms et en ce que la valeur de la mesure est déterminée pas plus tard qu'à 1,5 s après la fin du déplacement de la sonde.

**Claims**

1. An apparatus for recording $pO_2$ histograms in living tissue by means of a needle probe which carries a $pO_2$ measuring site in the distal zone and is movable axially by a drive to discrete places to be measured, the measuring site being connected to a measurement signal evaluator, characterised by the following features:

a) the outer diameter of the probe (111, 112) is over approximately 10 μm in the zone of the measuring site (213);

b) the drive (120) executes between the places for measurement (211, 212) steps greater than the distance between the measuring site and the probe apex plus a tissue-type-dependent distance (231) of the order of magnitude of probe diameter in the distal zone;

c) the time constant ($\tau_v$) of the response behaviour of the apparatus including the measuring site to $pO_2$ variations is at least approximately 30 times less than the average time constant ($T_g$) of the decrease of the interstitial tissue $pO_2$ after interruption of the oxygen supply;

d) the evaluator (140, 150) determines at the place of measurement a measurement value at a time ($T_4$) which is between about $3\tau_v$ and approximately $1/10\tau_g$ after the instant of termination of the advance movement ($T_2$), and

e) the step time ($T_2$) of the probe apex is at least one to two orders of magnitude less than the time constant ($\tau_g$) of the tissue $pO_2$ decrease.

2. An apparatus according to claim 1, characterised in that the ration $\tau_g/\tau_v$ is more than approximately 100.

3. An apparatus according to claim 1 and/or 2, characterised in that the probe diameter in the distal zone (211) is over approximately 100 μm.

4. An apparatus according to any of the previous claims, characterised in that the measuring site (213) is secured in the ground aperture of a metal cannula.

5. An apparatus according to any of the previous claims having a polarographic measuring site, characterised in that the measuring site is the ground surface (52) of a metal film (54) which is embedded between isolating media (55, 53) and which is covared by a diffusion permeable diaphragm (62).

6. An apparatus according to claim 5, characterised in that the film (54) is formed on a fibre core (55) and potted in a potting composition (53).

7. An apparatus according to claim 5 and/or 6, characterised in that the surface (60) of the metal film (54) is disposed below the ground surface (52) by an amount of the order of magnitude of film thickness.

8. An apparatus according to any of the previous claims for determination in human skeleton musculature, characterised in that the probe diameter is approximately 200 to 500 μm and the step width is over approximately 400 μm, $\tau_v$ is less than approximately 150 ms and the measurement value is ascertained at the latest by approximately 1.5 s after the end of the movement.

Fig. 1

0 104 493

Fig. 2a

Fig. 2 b

Fig. 3a

Fig. 3b

Fig. 3c

Fig. 3d

Fig. 3e

*Fig. 4*

1

57

56

55

54

53

52

51

213

111

100 μ

Fig. 5

Fig. 6